# FASCICULE DE BREVET EUROPEEN

(11) **EP 1 186 295 B1**
(45) Date de publication et mention de la délivrance du brevet: **26.07.2006**
(21) Numéro de dépôt: 01402270.1
(22) Date de dépôt: 31.08.2001
(51) Int. Cl.: A61K 31/35, A61K 36/00, A61K 35/74, A61P 5/00

(54) **Préparations pharmaceutiques contenant des extraits d'isoflavone de soja et des micro-organismes probiotiques**
Pharmazeutische Zubereitungen die Sojaisoflavonsextrakte und probiotische Mikroorganismen enthalten
Pharmaceutical preparations containing extracts of soy isoflavone and probiotic micro-organisms

(30) Priorité: 11.09.2000 FR 0011515
(43) Date de publication de la demande: 13.03.2002
(73) Titulaire: BOIRON, 69110 Sainte-Foy-Lès-Lyon (FR)
(72) Inventeur: Fabre, Pierre, 81106 Castres (FR); Belle, René, 81600 Rivieres (FR); Fabre, Bernard, 31450 Belberaud (FR)
(74) Mandataire: Denjean, Eric

(56) Documents cités:
- WO-A-00/03684
- WO-A-98/21946
- WO-A-99/07239
- PATENT ABSTRACTS OF JAPAN vol. 1998, no. 01, 30 janvier 1998 (1998-01-30) & JP 09 238647 A (YAKULT HONSHA CO LTD), 16 septembre 1997 (1997-09-16)
- HALL D. C.: "Nutritional influences on estrogen metabolism" APPLIED NUTRITIONAL SCIENCE REPORTS, vol. 451, janvier 2001 (2001-01), XP002167361

## Description

La présente invention se rapporte à des associations entre des isoflavones de soja et des micro-organismes probiotiques, et à leur utilisation en thérapeutique.

Le soja appartient à la famille des *Fabaceae.* Le fruit du soja est une gousse contenant des graines arrondies ou ovoïdes enfermées par deux, trois ou quatre. Ces graines de soja sont composées essentiellement de glucides, de protéines et de lipides. Les glucides qui représentent 15 à 25% de la matière sont constitués de sucres simples comme le saccharose, le raffinose et le stachyose, de pentosames et de galactosannes, mais très peu d'amidon. Les protéines sont abondantes puisqu'elles représentent 35 à 40% de la matière et sont constituées principalement d'albumines et de globulines. C'est cette richesse en protéines qui fait la valeur alimentaire des graines de soja. Les lipides correspondent à 15 à 20% de la matière et sont constitués principalement de glycérides d'acides gras saturés ou non saturés, d'un insaponifiable riche en phytostérols et d'une fraction lipidique polaire riche en phospholipides appelée lécithine.

Les isoflavones sont également un élément constitutif important des graines de soja, mais la teneur du soja en isoflavones est considérablement variable en fonction de l'espèce, de la maturité de la plante et des conditions de culture. Ces isoflavones sont des polyphénols hétérocycliques caractérisés par un enchaînement en C15 de type Ar-C₃-Ar arrangés selon un motif 1,2-diphénylpropanique. La structure générale est donnée dans la formule (1) ci-dessous:

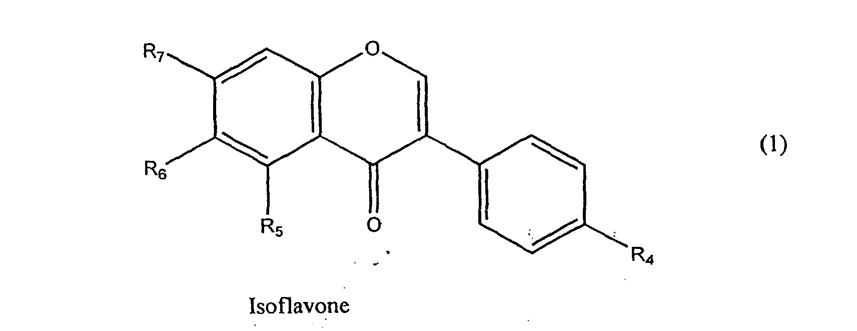

Dans la plante, les principales isoflavones, génistéine de formule (2) 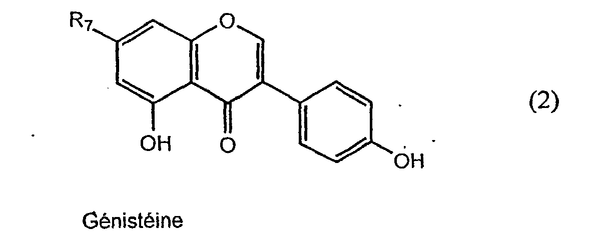 et daidzéine de formule (3) où R₇ = hydrogène, 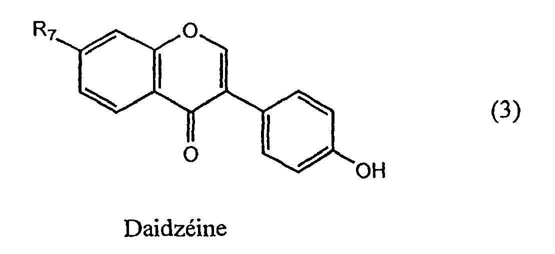 sont présentes sous différentes formes: glycosides, malonides, acétyles et aglycones, cette dernière forme étant minoritaire puisqu'elle ne représente que 1 à 2% des isoflavones totaux. Les conjugaisons ont lieu en position 7.

Wang H. *et al*. (*J. Agric*. *Food Chem.* (1994) **42,** n°8, pages 1666 à 1673) ont déterminé la présence de 2,275 mg d'isoflavones, par gramme de soja, sous toutes les formes citées précédemment, ce qui correspond à des teneurs de 43% en daidzéine et 57% en génistéine.

Tham D.M. *et al.* (*J*. *Clin. Endocrinol*. *Metab.* (1998) **83**, n°7, pages 2232 à 2235) ont titré deux espèces différentes à 1,005 mg/g et 4,031 mg/g avec 36% et 32% respectivement de daidzéine.

Patent abstract of Japan, vol. 1998 n°1, 30 janvier 1998, § JP 09238647 mentionne un aliment susceptible de prévenir le cancer et contenant des isoflavones obtenus par hydrolyse des isoflavones glycosides contenus dans du lait de soja par l'action de micro-organismes sur ledit lait de soja, lesdits micro-organismes appartenant au genre *Lactobacillus, Bifidobacterium, Streptococcus, Bacillus, Sacharomyces, Torulaspora* et Candida.

La demande internationale WO 00/03684 décrit des formulations à base de soja dont la concentration en isoflavones est supérieure à celle contenues dans le produit naturel. Ces formulations peuvent éventuellement comprendre un médicament, comme par exemple des traitements hormonaux de substitution, ainsi que différents autres éléments comme par exemple des enzymes de la digestion ou des vitamines, mais jamais il n'est fait mention de micro-organismes.

Ces compositions diminuent les effets de la ménopause, notamment les bouffées de chaleur (page 18 lignes 16 à 17 + page 19 ligne 11 à page 20 ligne 12).

La demande internationale WO 99/07239 décrit une combinaison de fructo-oligosaccharide, de farine de soja contenant des flavonoïdes et des graines de lin broyées contenant des lignanes, aptes à augmenter l'activité intestinale et à faciliter la conversion des phyto-oestrogènes en des formes utilisables par les intestins, ainsi que leur absorption. Dans cette combinaison, les fructo-oligosaccharides sont utilisés pour leurs effets prébiotiques, c'est-à-dire par leur capacité à augmenter de manière sélective la croissance des bifidobactéria et des lactobacilles qui augmentent la production d'acide dans les intestins. Ces compositions sont utiles pour traiter les symptômes de la ménopause.

La demande internationale WO 98/21945 décrit des compositions qui sont susceptibles de diminuer les manifestations du SPRT, (Symptoms of Persistent Reproductive Transition), parmi lesquelles les bouffées, de chaleur; ces compositions contiennent des phyto-oestrogène, notamment issus du soja associé à des sucres et des cholines.

En fait, de façon générale, on retient la valeur de 2 mg d'isoflavones par gramme de soja dans la graine sèche.

La structure des isoflavones présente des homologies avec celles des oestrogènes. Ainsi, sans être des stéroïdes, la génistéine et la daidzéine possèdent des groupement hydroxyles en position 7 et 4' dans une configuration stéréochimique analogue à celle des hydroxyles de la molécule d'oestradiol de formule(4).

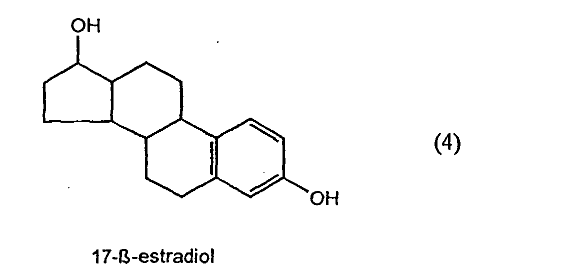

Du fait de cette homologie structurale avec l'oestradiol, et plus particulièrement du fait de la distance et de l'angle dièdre entre les deux groupement hydroxyles, les isoflavones ont une activité oestrogénique. Elles peuvent en effet se lier comme l'oestradiol sur les récepteurs de type II (β) (Bennetau-Pelissero *Lettre Scientifique de l'Institut Français pour la Nutrition* (1997) **47**), cependant, cette activité est au moins 500 fois plus faible que celle de l'oestradiol.

En effet, si l'on fixe à 100 l'activité de l'oestradiol, alors la daidzéine a une activité de 0,013, la génistéine une activité de 0,084 et l'équol, dérivé métabolique de la daidzéine, a une activité de 0,61 (Markiewicz L., *J. Steroid Biochem. Molec. Biol.* (1993) **45**, pages 399 à 405).

Le soja est consommé par les populations asiatiques, plus particulièrement sous des formes fermentées comme le miso ou le tempeh. L'observation de ces populations, qui ont donc une alimentation très riche en phyto-oestrogènes et consomment entre 20 et 150 mg d'isoflavones par jour Murkies A. *et al*. (*J. Clin. Endocrinol. Metab*.(1998) **83**, n°2, pages 297 à 303), a révélé des différences par rapport aux populations occidentales sur le plan de la santé. Ces populations présentent:
- 4 à 10 fois moins de probabilités de décéder d'un cancer hormono-dépendant (sein, endomètre, prostate et colon),
- moins de symptômes corrélés à des désordres hormonaux au moment de la ménopause (ostéoporose, bouffées de chaleur), et
- moins de maladies coronariennes et du coeur.

Du fait de leur homologie structurale avec les oestrogènes, les isoflavones sont souvent utilisées en médecine, principalement en gynécologie médicale. Ainsi, Wilcox C. *et al.* (*BMJ* (1990) **24**, pages 1757 à 1761) et Washburn S.A. *et al*. (Third international conference on phyto-oestrogens (1995) Little Rock, USA) ont montré respectivement, dans des études contrôlées contre placebo, une baisse importante et significative des bouffées de chaleur lors d'ingestion de soja à raison de 40 g/jour pendant 6 semaines. Albertazzi P. *et al*. *(Am. J. Obstet. Gynecol.* (1998) **91**, pages 6 à 11) ont confirmé cet effet avec 60 g de soja (soit 76 mg d'isoflavones) par jour dans une étude randomisée chez plus de 100 femmes ménopausées. Ces études démontrent par conséquent l'effet des phyto-oestrogènes sur les bouffées de chaleur.

En outre, chez la femme, des études ont été réalisées par Valente M. *et al.* (*Calcif. Tissue Int*. (1994) **54**, pages 377 à 380) avec l'ipriflavone, prodrogue synthétique de la daidzéine, à raison de 500 à 600 mg par jour et ont montré chez 40 femmes ménopausées souffrant d'ostéoporose, un gain osseux au niveau de L1-L5 avec un retour à la normale en 12 mois. Gambacciani M. *et al.* (*Bone and mineral,* 26, pages 19 à 26) a montré que l'on peut également éviter les pertes osseuses chez les femmes sous agonistes de LHRH (*Luteinizing Hormone Releasing Hormone).* Blair *et al.* (*J. Cell. Biochem.,* 1996, 61, pages 629 à 637) expliquent cette action en mettant en évidence une inhibition de l'activité ostéoclastique par la génistéine.

Dans le cas des cancers hormonaux dépendants, Barnes S. et *al*. (*Diet and cancer: markers*, *prevention and treatment* (1994) Plenum Press, pages 135 à 147) ont montré une réduction du nombre et de la taille des tumeurs. Des études fondamentales réalisées par Pagliacci N.C. *et al*. (*European J. of Cancer* (1994) **30A** (11) pages 1675 à 1682) ont montré l'inhibition de cellules cancéreuses mammaires humaines par les phyto-oestrogènes, alors que Wutke W. (*in Proceedings of* 2^{è} Congrès de la société européenne de gynécologie (1997)) a mis en évidence l'inhibition de l'expression des gènes responsables de la croissance de cultures cellulaires. Enfin, l'étude de Lee H.P. *et al.* (*Lancet* (1991) **337**, n°18, pages 1197 à 1200) a montré que le risque de cancer du sein chute avec une alimentation riche en soja chez la femme ménopausée.

L'intestin grêle est le lieu privilégié de l'absorption des isoflavones car la surface de contact est grande, et le réseau sanguin dense. Cependant, seules les formes aglycones (génistéine et daidzéine) sont absorbées car les glycosides ont une trop grande hydrophilie, et un trop grand poids moléculaire.

Il a été démontré que seules les formes aglycones ont une activité oestrogénique chez les animaux (Tham D.M. *et al.* déjà cité et Moreau L. *et al*. (*Références en gynécologie obstétrique* (1999), pages 229 à 234, Springer-Verlag). La biotransformation des isoflavones glycosylées en équol, génistéine et daidzéine est donc nécessaire pour l'observation des effets bénéfiques du soja.

Chang Y.C. *et al.* (*J. Nat. Prod.* (1995) **58,** n°12, pages 1892 à 1896) ont montré que cette transformation nécessaire des isoflavones glycosylées (daidzine et génistine) en daidzéine, génistéine et équol est effectuée par la microflore intestinale.

Pour utiliser les isoflavones, la flore intestinale comprend donc des bactéries capables d'hydrolyser les liaisons ß 1-4 et de libérer ainsi les formes aglycones. Ces micro-organismes sécrètent des enzymes telles que des β-glucosidases, mais cette déglycosylation est également effectuée en partie par simple hydrolyse grâce à l'acide chlorhydrique gastrique.

Enfin, les molécules déjà absorbées, et donc conjuguées, peuvent être déconjuguées et réabsorbées grâce aux enzymes ß-gluçuronidase et arylsulfurase.

La diversité de la microflore intestinale joue un rôle important dans les voies de métabolisation et la biodisponibilité des isoflavones, notamment les *Lactobacilles,* les *Bactéroïdes* et les *Bifidobactéries* (Xu X. *et al*. (1995) *Bioavailability of soybean isoflavons dépends upon gut microflora in women I,* Nutro **125,** pages 2307 à 2315).

En général, la conversion est efficace, et seulement 14 à 43% des isoflavones ingérées sont retrouvées intactes dans l'urine.

Toutefois, il existe une grande variété métabolique individuelle et Moreau *et al*. *(Références en gynécologie obstétrique* déjà cité) ont montré que 30% de la population n'a pas la flore capable de métaboliser l'équol - forme aglycone la plus efficace par rapport aux effets oestrogéniques, ce qui rend difficile un traitement par les isoflavones.

Les probiotiques sont des "organismes vivants qui, ingérés en quantité suffisante, exercent des effets bénéfiques sur la santé allant au delà des vertus nutritionnelles inhérentes". Les bienfaits sur la santé ne se limitent pas à l'amélioration de l'équilibre microbien au niveau de l'intestin, mais peuvent recouvrir d'autres effets. Ainsi, pour les bactéries lactiques, cinq types d'effets sont observés:
- Effets sur le tube digestif: amélioration de la digestion du lactose chez les personnes déficientes en lactase, prévention des troubles intestinaux, adhérence aux cultures de cellules intestinales, stimulation de l'immunité intestinales dans les modèles animaux, stabilisation de la maladie de Crohn et régulation de la motilité intestinale.
- Modification de la microflore: équilibre des bactéries intestinales, augmentation des bactéries fécales, diminution de l'activité des enzymes fécales, diminution de la mutagénicité fécale, soulagement des symptômes de l'intolérance au lactose et colonisation du tube digestif.
- Effet sur la diarrhée: prévention et/ou traitement des diarrhées aiguës et à rotavirus, réduction des diarrhées à rotavirus, prévention de la diarrhée associée aux antibiotiques, traitement des diarrhées récidivantes à *Clostridium difficile* et traitement des diarrhées persistantes chez l'enfant.
- Effets systémiques: stimulation de l'activité phagocytaire, stimulation de la production d'interféron γ par les cultures de cellules mononucléaires du sang humain, réduction de l'hypertension dans les modèles animaux et humains, effets bénéfiques sur la cancer superficiel de la vessie et soulagement des symptômes cliniques chez les enfants atteints de dermatite optique.
- Effets physiologiques: production de bactériocines et effet antagoniste contre *Helicobacter pylori.*

Les Bifidobactéries, quant à elles, améliorent le transit gastro-intestinal, diminuent le risque du cancer du côlon et possèdent une activité antimicrobienne et des effets immunomodulateurs, alors que *Lactobacillus acidophilus* possède des effets hypocholéstérolémiant, antimicrobien et immunomodulateur, tout en prévenant le cancer du côlon.

En conséquence, il apparaît nécessaire de pallier la faible activité oestrogénique des isoflavones et d'améliorer leur activité chez les patientes présentant des symptômes pré et/ou ménopausaux et plus particulièrement chez les patientes peu ou pas répondeuses.

Or, les inventeurs ont trouvé de manière surprenante que certains micro-organismes probiotiques associés aux isoflavones pouvaient résoudre ce problème.

La présente invention a en conséquence pour objet des préparations pharmaceutiques comprenant des extraits titrés en isoflavones de soja et des micro-organismes probiotiques vivants, comme produit de combinaison pour une administration simultanée, séparée ou étalée dans le temps utile dans le traitement des symptômes liés à la ménopause.

Dans un mode particulier de réalisation, l'extrait d'isoflavones est titré de 10 à 50% en isoflavones.

Dans un autre mode particulier de réalisation, les micro-organismes probiotiques sont choisis dans le groupe constitué par *Enterococcus faecium, Propionibacterium acidipropionici, Lactobacillus acidophilus*, *Lactobacillus brevis, Lactobacillus plantarum*, *Lactobacillus* casei, *Lactobacillus rhamnosus*, Pediococcus *acidilactici* et *Streptococcus thermophilus* et les mélanges de ces micro-organismes.

Dans un mode particulièrement avantageux de réalisation de l'invention, les préparations comprennent l'extrait d'isoflavones titré entre 10 et 50% en isoflavones et les micro-organismes probiotiques sont choisis dans le groupe constitué par *Enterococcus faecium*, *Propionibacterium acidipropionici*, *Lactobacillus acidophilus* et *Lactobacillus brevis.*

Au sens de la présente invention, on entend par extrait titré, en isoflavones de soja un extrait préparé comme suit: les graines de soja broyées sont extraites par une solution hydro-alcoolique. Après élimination de l'alcool, la solution aqueuse est délipidée à l'aide d'un solvant organique puis purifiée sur colonne sur une résine absorbante.

Les micro-organismes probiotiques sont choisis sur la base de leurs propriétés biologiques et leurs capacités à métaboliser les isoflavones.

Ces micro-organismes peuvent être utilisés sous forme de culture disponibles dans le commerce, sous forme de suspension microbienne ou de préférence sous forme d'un lyophilisat préparé dans des conditions classiques connues de l'homme du métier.

Dans un mode de réalisation particulièrement avantageux de l'invention, ces micro-organismes sont présents à raison de 50.10⁹ à 1.10⁶ bactéries par dose unitaire et l'extrait d'isoflavone est présent à raison de 100 à 300 mg par unité de prise.

Les préparations, conformes à l'invention, sont particulièrement utiles comme médicament, notamment pour les traitements des symptômes liés à la ménopause, particulièrement contre les bouffées de chaleur.

Les préparations selon l'invention comprenant l'extrait de soja et les micro-organismes peuvent être présentées sous toute forme sèche ou fluide habituellement utilisée, notamment sous forme de tablettes, de comprimés, de capsules, de gélules, ou de solutions buvables en association avec tout excipient pharmaceutiquement acceptable.

L'extrait d'isoflavones et les micro-organismes probiotiques peuvent être également administrés sous forme de doses unitaires séparées, notamment des tablettes, des comprimés, des capsules, des gélules ou des solutions buvables qui peuvent être administrées séparément ou simultanément ou de manière étalée dans le temps.

Les préparations selon l'invention permettent une métabolisation des formes glycosylées en aglycones actives dès la première absorption, au niveau du duodénum, zone d'absorption maximale du tube digestif.

Les exemples qui suivent illustrent l'invention sans toutefois la limiter.

### Exemple 1: Exemples de formulation

### Formulation 1:

- extrait de soja titré à 15% en isoflavones, 250 mg dans une gélule de taille 1,
- lyophilisat de *Lactobacillus acidophilus*, 10⁹ bactéries dans une gélule de taille 1
prendre 1 gélule de chaque simultanément par jour.

### Formulation 2:

- extrait de soja titré à 15% en isoflavones, 100 mg,
- lyophilisat de *Lactobacillus acidophilus*, 10⁶ bactéries dans même une gélule de taille 1
prendre 2 gélules par jour matin et soir ou 2 le matin ou 2 le soir.

### Formulation 3:

- extrait de soja titré à 40% en isoflavones, 200 mg formulé dans un comprimé,
- lyophilisat de *Lactobacillus brevis,* 10⁹ bactéries dans une gélule de taille 1
prendre une gélule de chaque simultanément par jour.

### Formulation 4:

- extrait de soja titré à 10% en isoflavones, 200 mg dans une capsule,
- lyophilisat de *Enterococus faecium,* 10⁹ bactéries dans une capsule
prendre 4 capsules de chaque simultanément par jour.

### Formulation 5:

- solution hydro-alcoolique à 2% en extrait de soja titré à 50% en isoflavones,
- suspension microbienne de *Lactobacillus acidophilus* à 10⁸ germes/g,
prendre à l'aide d'une cuillère à mesure 10 ml de chacune des solutions par jour.

### Exemple 2: Sélection des micro-organismes métabolisateurs des isoflavones.

### 1) Mode opératoire:

Des micro-organismes, présents dans la flore fécale et capables de métaboliser la génistine et la daidzine respectivement en génistéine et daidzéine, ont été mis en culture durant 24, 48 et 72 heures dans un milieu nutritif contenant: peptone de viande 10 g/l, extrait de viande 10 g/l, extrait de levure 5 g/l, glucose 20 g/l, acétate de sodium 5 g/l, tween80 1 g/l, phosphate dipotassique 2 g/l, citrate d'ammonium 2 g/l, sulfate de magnésium 0,2 g/l, sulfate de manganèse 0,05 g/l, et un extrait de soja. A chacun de ces temps, un prélèvement des cultures microbiennes est effectué, dilué de moitié dans du méthanol puis filtré. La solution méthanolique est analysée en chromatographie liquide haute performance. La daidzine, génistine, daidzéine et génistéine sont dosées par chromatographie liquide haute performance dans l'extrait de départ et dans les milieux métabolisés par les différents micro-organismes. On calcule ainsi les teneurs des formes aglycones et glycosylées de l'extrait métabolisé par rapport aux formes glycosylées (100%) dans l'extrait initial.

### 2) Résultats.

Ils sont rassemblés dans le tableau 1 ci-après.

**TABLEAU 1**

| | *Enterococcus faecium* | *Propionibacterium acidipropionici* | *Lactobacillus acidophilus* | *Lactobacillus brevis* | *Lactobacillus plantarum* | Pediococcus acidilactici | Streptococcus thermophilus | Lactobacillus casei | Lactobacillus rhamnosus |
|---|---|---|---|---|---|---|---|---|---|
| Génistine | 5,5% | 12% | 6,5% | 2% | 53% | 63% | 73% | 85% | 5% |
| Daidzine | 0,1% | 33% | 16% | 16% | 75% | 77% | 65% | 80% | 26% |
| Génistéine | 80% | 72% | 83% | 81% | 44% | 30% | 25% | 13% | 85% |
| Daidzéine | 85% | 57 % | 84% | 82% | 22% | 23% | 33 % | 18% | 71% |

Ces souches de micro-organismes métabolisent environ 80% des formes glycoconjuguées des isoflavones en formes aglycones qui sont facilement absorbées dans l'intestin; *l'Enterococcus faecium*, *Propionibacterium acidipropionici*, *Lactobacillus acidophilus* et *Lactobacillus brevi* s'avèrent les souches les plus actives.

### Exemple 3: Evaluation clinique

### 1) Modalités de l'étude:

Elle a été mise en oeuvre dans l'objectif de tester l'efficacité de l'association soja-probiotique avec le critère d'efficacité bouffée de chaleur dans l'indication ménopause avec 2 groupes de femmes ménopausées recevant un extrait de soja associé ou non au probiotique.

Cette étude s'est déroulée pendant 3 mois avec une période de pré-inclusion d'un mois.

Un groupe de 50 femmes ménopausées a été traité 1 fois par jour avec une gélule contenant 10⁹ bactéries/g de *Lactobacillus acidophilus*. Un autre groupe de 50 femmes ménopausée a reçu 1 fois par jour une gélule de 250 mg d'extrait de soja à 15% en isoflavones et une gélule placebo.

Chaque sujet a noté dans un carnet d'auto-évaluation le nombre de bouffées de chaleur quotidienne et leur intensité selon un barème préétabli.

### 2) Résultats:

Cette étude montre le rôle positif important des probiotiques dans la régularité et l'intensité de la réponse aux phyto-oestrogènes, puisque les préparations selon l'invention permettent aux femmes ayant reçu l'extrait de soja associé au probiotique de constater une diminution du nombre et de l'intensité des bouffées de chaleur supérieures à celles ayant reçu l'extrait de soja seul.

## Revendications

1. Utilisation d'une préparation pharmaceutique comprenant des extraits titrés en isoflavones de soja et des micro-organismes probiotiques vivants, comme produit de combinaison pour une administration simultanée, séparée ou étalée dans le temps pour la préparation d'un médicament, utile dans le traitement des bouffées de chaleur liées à la ménopause, **caractérisée en ce que** les micro-organismes probiotiques sont choisis dans le groupe constitué par *Enterococcus faecium*, *Propionibacterium acidipropionici*, *Lactobacillus acidophilus, Lactobacillus brevis*, *Lactobacillus plantarum*, *Lactobacillus* casei, *Lactobacillus rhamnosus*, Pediococcus *acidilactici* et *Streptococcus thermophilus* et les mélanges de ces micro-organism

2. Utilisation selon la revendication 1, **caractérisée en ce que** l'extrait d'isoflavones est titré entre 10 et 50% en isoflavones.

3. Utilisation selon les revendications 1 et 2, **caractérisée en ce que** l'extrait d'isoflavones est titré entre 10 et 50% en isoflavones et les micro-organismes probiotiques sont choisis dans le groupe constitué par *Enterococcus faecium, Propionibacterium acidipropionici*, *Lactobacillus acidophilus* et *Lactobacillus brevis.*

4. Utilisation selon l'une quelconque des revendications 1 à 3, **caractérisée en ce que** les micro-organismes probiotiques sont utilisés sous forme d'une suspension microbienne ou d'un lyophilisat.

5. Utilisation selon l'une quelconque des revendications 1 à 4, **caractérisées en ce que** les micro-organismes probiotiques sont présents à une concentration comprise entre 50.10⁹ et 1.10⁶ bactéries par unité de prise.

6. Utilisation selon l'une quelconque des revendications 1 à 5, **caractérisée en ce que** l'extrait d'isoflavones est présent à raison de 100 à 300 mg par unité de prise.

## Claims

1. Use of a pharmaceutical preparation containing extracts titrated with soy isoflavones and live probiotic micro-organisms, as a combination product for simultaneous, separate or time-released administration for the preparation of a medicinal product, useful in the treatment of hot flashes related to menopause, **characterised in that** the probiotic micro-organisms are selected from the group comprising *Enterococcus faecium, Propionibacterium acidipropionici, Lactobacillus acidophilus, Lactobacillus brevis, Lactobacillus plantarum, Lactobacillus casei, Lactobacillus rhamnosus, Pediococcus acidilactici* and *Streptococcus thermophilus* and mixtures of these micro-organisms.

2. Use as claimed in claim 1, **characterised in that** the isoflavone extract is titrated between 10% and 50% isoflavones.

3. Use as claimed in claim 1 and 2, **characterised in that** the isoflavone extract is titrated between 10% and 50% isoflavones and the probiotic micro-organisms are selected from the group comprising *Enterococcus faecium, Propionibacterium acidipropionici, Lactobacillus acidophilus* and *Lactobacillus brevis.*

4. Use as claimed in any of claims 1 to 3, **characterised in that** the probiotic micro-organisms are used in the form of a microbial suspension or lyophilizate.

5. Use as claimed in any of claims 1 to 4, **characterised in that** the probiotic micro-organisms are present in a concentration between 50.10⁹ and 1.10⁶ bacteria per dosage unit.

6. Use as claimed in any of claims 1 to 5, **characterised in that** the isoflavone extract is present at a rate of 100 to 300 mg per dosage unit.

## Patentansprüche

1. Verwendung eines pharmazeutischen Präparats, umfassend Soja-Extrakte mit eingestelltem Gehalt an Isoflavonen und lebende probiotische Mikroorganismen, als Kombinationsprodukt zur gleichzeitigen, separaten oder zeitversetzten Verabreichung für die Herstellung eines Medikaments, das bei der Behandlung von Hitzewallungen in Verbindung mit der Menopause brauchbar ist, **dadurch gekennzeichnet, dass** die probiotischen Mikroorganismen ausgewählt sind aus der Gruppe bestehend aus *Enterococcus faecium, Propionibacterium acidipropionici, Lactobacillus acidophilus, Lactobacillus brevis, Lactobacillus plantarum, Lactobacillus casei, Lactobacillus rhamnosus, Pediococcus acidilactici* und *Streptococcus thermophilus* und Mischungen dieser Mikroorganismen.

2. Verwendung nach Anspruch 1, **dadurch gekennzeichnet, dass** der Extrakt der Isoflavone auf einen Gehalt an Isoflavonen zwischen 10 und 50% eingestellt ist.

3. Verwendung nach den Ansprüchen 1 und 2, **dadurch gekennzeichnet, dass** der Extrakt der Isoflavone auf einen Gehalt an Isoflavonen zwischen 10 und 50% eingestellt ist und die probiotischen Mikroorganismen ausgewählt sind aus der Gruppe bestehend aus *Enterococcus faecium, Propionibacterium acidipropionici, Lactobacillus acidophilus* und *Lactobacillus brevis.*

4. Verwendung nach einem der Ansprüche 1 bis 3, **dadurch gekennzeichnet, dass** die probiotischen Mikroorganismen als mikrobielle Suspension oder als Lyophilisat eingesetzt werden.

5. Verwendung nach einem der Ansprüche 1 bis 4, **dadurch gekennzeichnet, dass** die probiotischen Mikroorganismen in einer Konzentration zwischen 50. 10⁹ und 1-10⁶ Bakterien pro Einnahmeeinheit vorhanden sind.

6. Verwendung nach einem der Ansprüche 1 bis 5, **dadurch gekennzeichnet, dass** der Extrakt der Isoflavone in einer Menge von 100 bis 300 mg pro Einnahmeeinheit vorhanden ist.
